Europäisches Patentamt

European Patent Office

Office européen des brevets

(11) Publication number: **0 248 627**
**A2**

# (12) EUROPEAN PATENT APPLICATION

(21) Application number: **87304839.1**

(22) Date of filing: **01.06.87**

(51) Int. Cl.4: **A61B 5/04**

(30) Priority: **05.06.86 GB 8613687**

(43) Date of publication of application:
**09.12.87 Bulletin 87/50**

(84) Designated Contracting States:
**DE FR GB IT**

(71) Applicant: **Rocket of London Limited**
**Imperial Way**
**Watford WD2 4XX(GB)**

(72) Inventor: **Meredith, Hadyn Gwyn**
**18 Farnleys Mead**
**Lymington Hampshire(GB)**
Inventor: **Fuller, Peter Brian**
**Coxhill Cottage**
**Coxhill Boldre Lymington Hampshire(GB)**

(74) Representative: **Pattullo, Norman et al**
**Ian G. Murgitroyd and Company Mitchell**
**House 333 Bath Street**
**Glasgow G2 4ER Scotland(GB)**

(54) **Instrument for monitoring foetal heart rate.**

(57) An instrument for monitoring foetal heart rate having a head member (2) for application to the foetal scalp, the head member (2) having two jaws (24,25) which are hingedly connected to one another, the first jaw (24) having an arcuate electrically conductive needle (9) extending therefrom and movable therewith for penetration of the foetal scalp. A wire (6) extends from the needle (9) to a terminal for connection to monitoring equipment. A second wire (7) is insulated from the first (6) and extends between an exposed electrode sheath (11), for contacting the mother in use, and a terminal for connection to monitoring equipment. The instrument also has actuating means, in the form of a telescopic arrangement of elongate members (1,15), for moving the jaws (24,25) apart. The jaws (24,25) are moved back to a preselected attitude by the bias provided by a resilient membrane (27).

FIG.2

## Instrument for Monitoring Foetal Heart Rate

This invention relates to an instrument especially but not exclusively for monitoring foetal heart rate.

During delivery monitoring of foetal heart rate is routine as it helps provide a complete birth record. However, the monitoring is especially important for babies at risk, as heart rate is a good indicator as to the condition of the child and the instrument therefore provides an early warning system for babies in distress.

The first successful device for monitoring foetal heart rate took the form of an insulated suture clip which could be attached to the foetal head and complete an electrical circuit with a second electrode in contact with the mother.

Later inventions included providing the instrument's leading end with a helical electrode or pair of electrodes, which attached to the baby's head by rotation of a handle which was attached to the electrode. Over-rotation could in some cases cause the baby's scalp to be traumatised, and result in difficult removal of the electrode.

Another device included a single arcuate needle electrode which was movable to emerge from or retract into a housing forming the active head of the device.

According to the present invention there is provided an instrument for monitoring foetal heart rate comprising a body having a head member for application to the foetal scalp, an electrically conductive needle extending from the head member for penetration of the foetal scalp, a first electrical conductor extending from the needle for connection to monitoring equipment, a second electrical conductor for connection to monitoring equipment, said second conductor being insulated from the first conductor and extending from a terminal external of the body for contacting the mother in use, characterised in that the head member is flexible and the needle movable therewith, and actuating means are provided for flexing the head member.

Preferably, biassing means are provided on the head member, to bias said head member towards a preselected attitude. The biassing means may for example comprise a resilient membrane which engages the head member and is distorted when the head member is moved from said preselected attitude.

Preferably, the actuating means is in the form of a telescopic arrangement of members, the members being connected with different regions of the head member for flexing said head member.

Preferably, the head member comprises first and second portions which are hingedly connected to one another.

Preferably, the needle is arcuate and centred on the axis of the hinge connection between the first and second portions of the head member.

Embodiments of the present invention will now be described, by way of example, with reference to the accompanying drawings, in which:

Fig. 1 is a back view of a first embodiment of an instrument according to the present invention;

Fig. 2 is a side view of the instrument of Fig. 1;

Fig. 3 is a detail of Fig. 2 with the head portion in an open position;

Fig. 4 is a perspective view of the front face of the head portion of the instrument of Figs. 1 and 2;

Fig. 5 is a perspective view of the back face of the head portion of the instrument of Figs. 1 and 2;

Fig. 6 is a perspective view of the trailing end of the instrument of Figs. 1 and 2;

Fig. 7 is a sectional view corresponding to Fig. 6 with electrical wires missing;

Fig. 8 is a side view of a second embodiment of an instrument according to the present invention;

Fig. 9 is a front elevation of the instrument of Fig. 8;

Fig. 10 is an enlarged view of the head portion of the instrument of Fig. 8 shown in a sectional view;

Fig. 11 is a back elevation of the head portion shown in Fig. 10;

Fig. 12 is a back elevation of a lower jaw of the head portion shown in Fig. 10;

Fig. 13 is a plan view of the lower jaw viewed on A of Fig. 12;

Fig. 14 is as sectional view of the lower jaw on X-X of Fig. 12;

Fig. 15 is an end elevation of the lower jaw viewed on B on Fig. 14;

Fig. 16 is a side elevation of a top jaw of the head portion shown in Fig. 10;

Fig. 17 is a plan view of the top jaw viewed on A of Fig. 16;

Fig. 18 is a back elevation of the top jaw of Fig. 16;

Fig. 19 is an enlarged back view of the trailing end of the instrument of Fig. 9;

Fig. 20 is a sectional side view of the plunger shown in Fig. 19; and,

Fig. 21 is a front view of slider tube of Fig. 19.

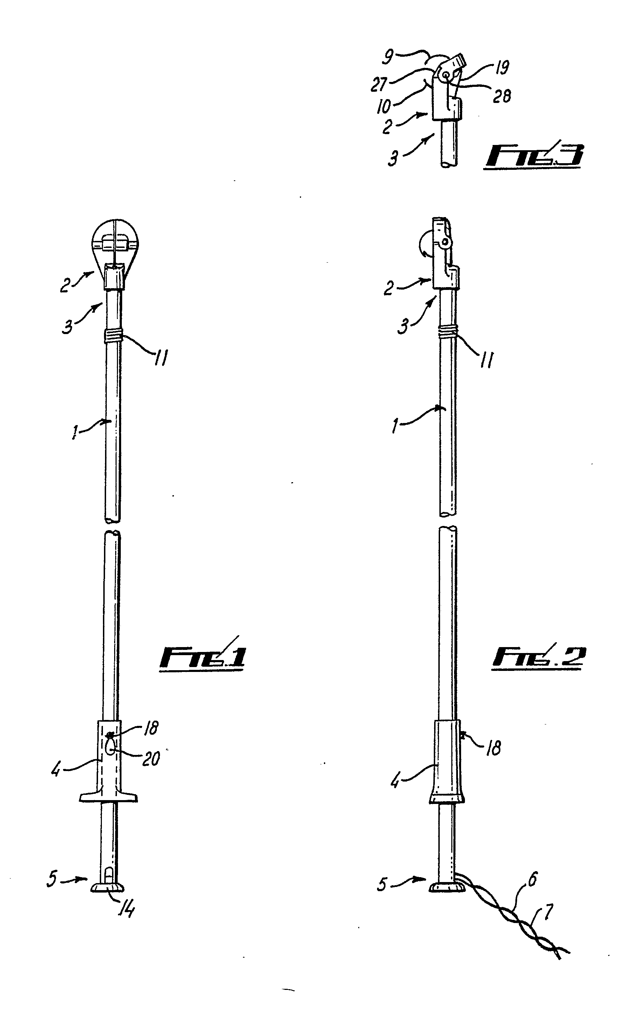

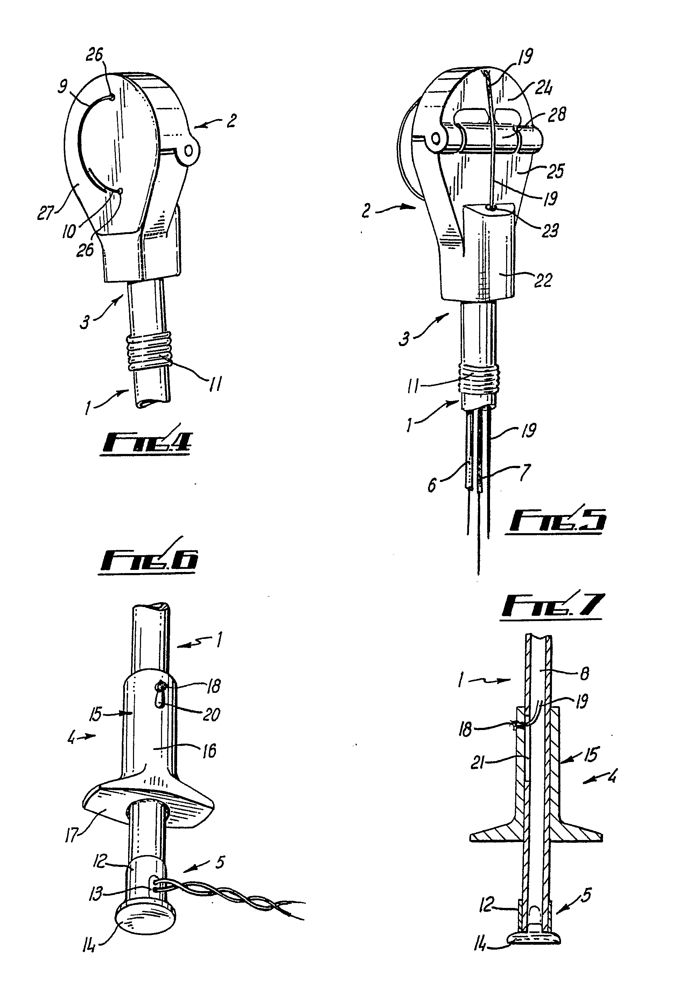

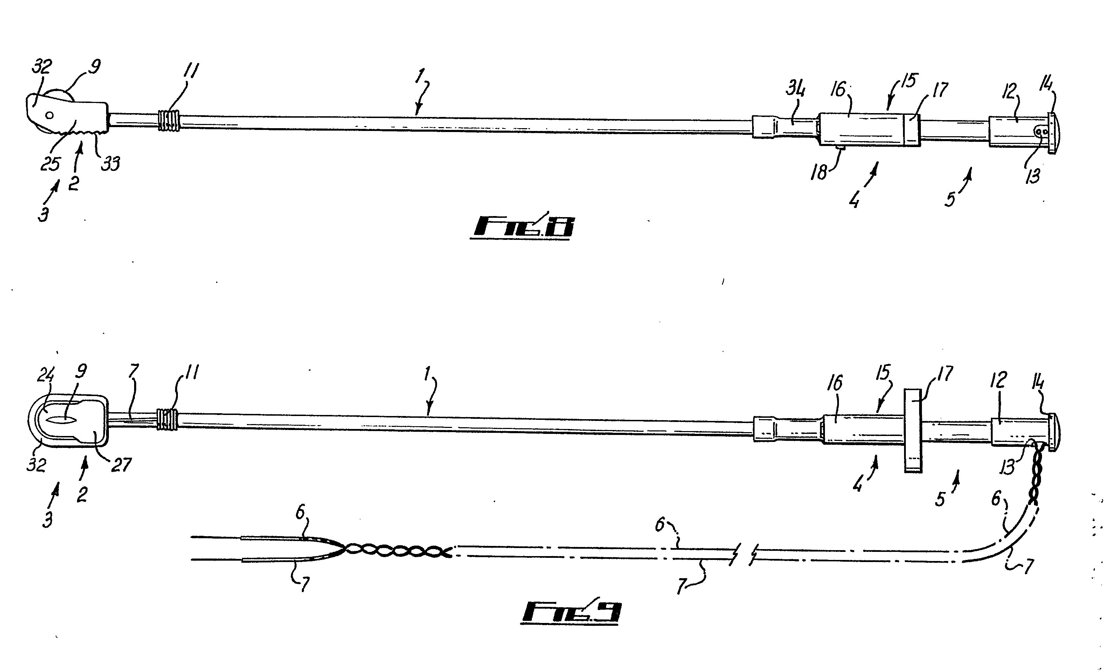

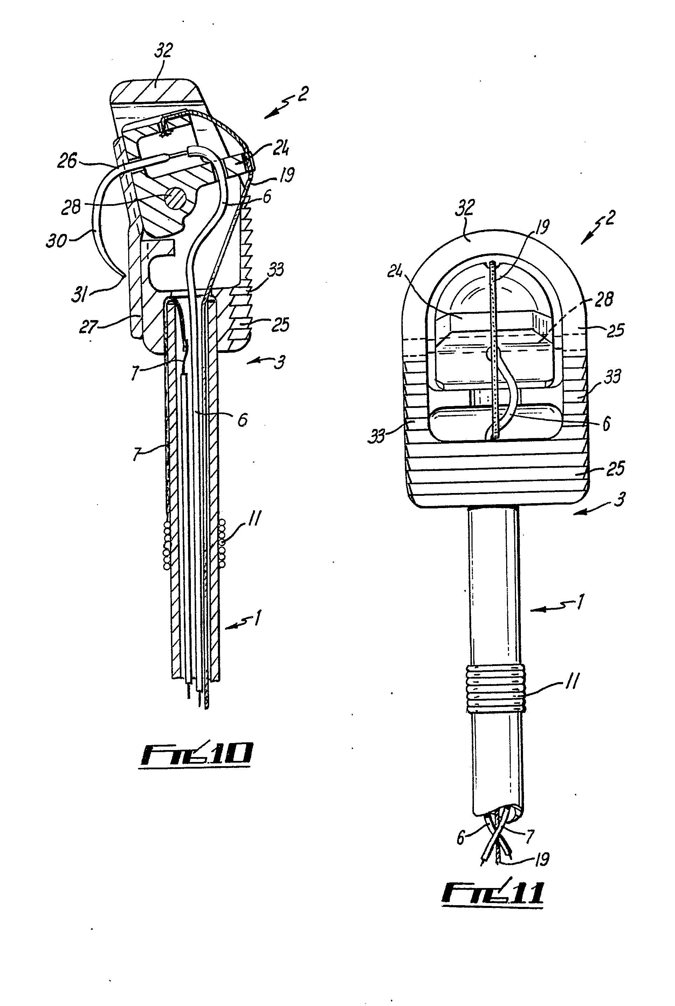

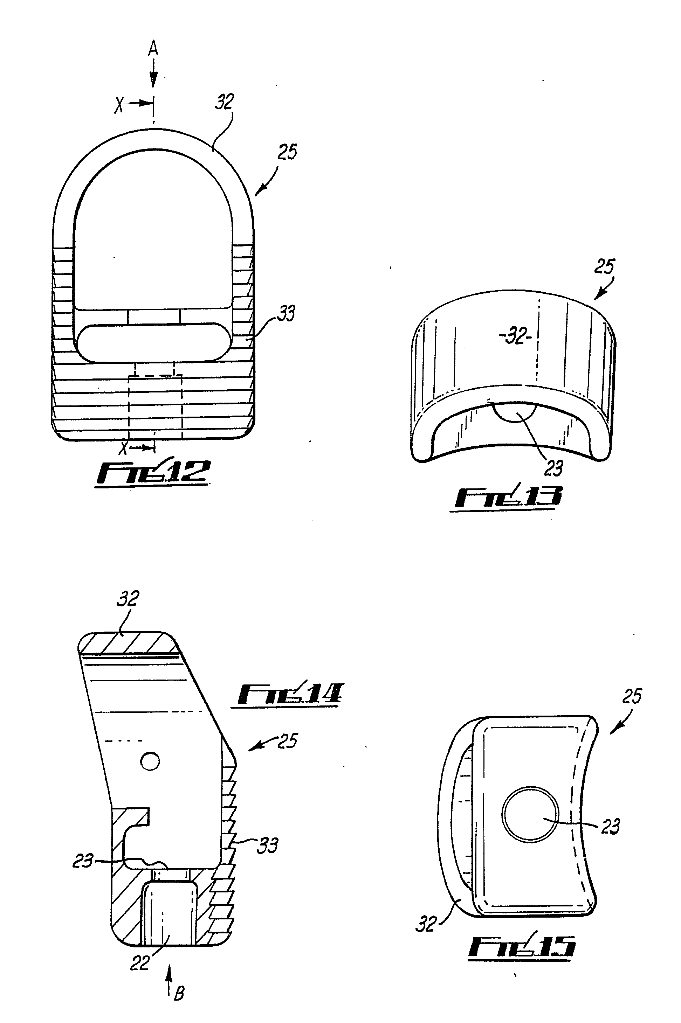

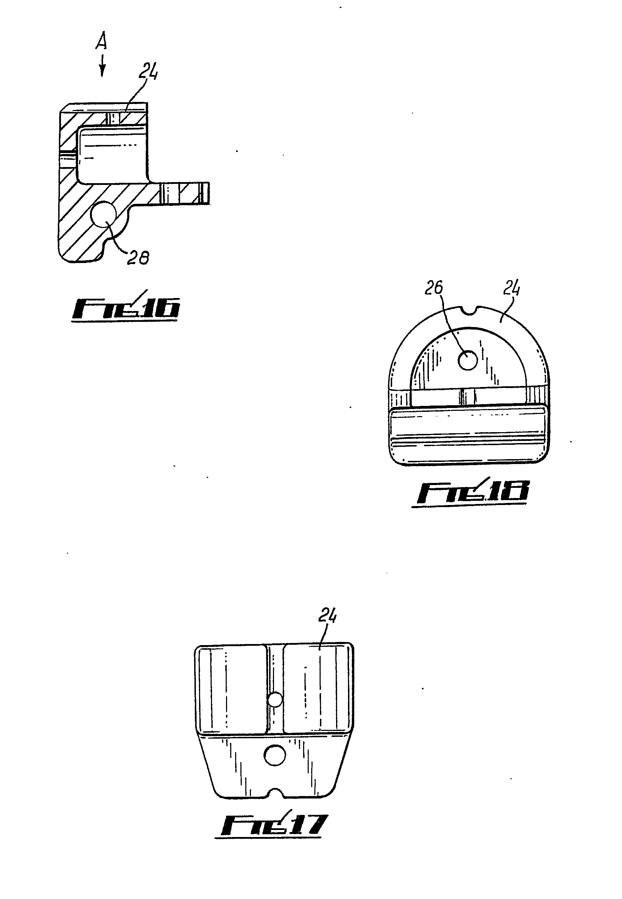

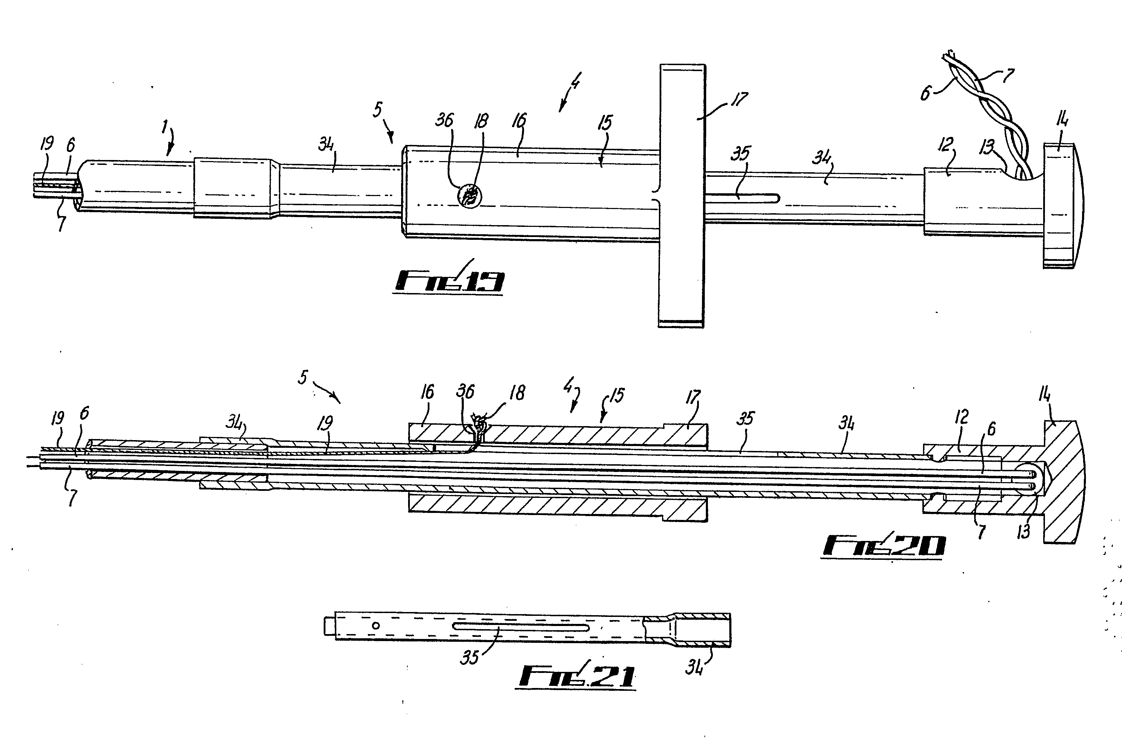

Referring to Figs 1 to 7 of the drawings, the instrument of this embodiment of the invention has a tubular main shaft 1 of flexible plastics with a head portion 2 at its leading end 3 and a plunger 4 at its trailing end 5. A pair of mutually insulated electrical wires 6, 7 are carried within the bore 8 of the main shaft 1, one of the wires 6 connecting to a pair of electrodes 9, 10 of arcuate form in the head portion 2, the other wire 7 connecting to an electrode sheath 11 of coiled wire on the exterior of the main shaft 1. The wires 6, 7 emerge from the bore 8 at the trailing end 5 through a small bite (not shown), this end being encircled by a tube of plastic 12 with corresponding bite 13 and its cut end being plugged with a rubber stopper 14.

The main shaft 1 is slidable within a handle 15 of the plunger 4. The handle 15 consists of a tube 16 coaxial with the main shaft 1 and carries a pair of wings 17 at its base. A knotted end 18 of a cord 19, which is carried alongside the wires 6, 7 in the bore 8 of the main shaft 1, is affixed to the handle 15, protruding through a key-hole-shaped aperture 20 in the handle 15 and a corresponding slot 21 in the main shaft 1.

The base of the head portion 2 has a cylindrical mould 22 which receives the leading end 3 of the main shaft 1. The cord 19 emerges through a circular aperture 23 at the top of the cylindrical mould 22 and attaches to a top jaw 24 of the head portion 2. The arcuate electrodes. 9, 10 extend rigidly from the top jaw 24 and a bottom jaw 25 of the head portion 2 respectively, protruding through small perforations 26 in a rubber membrane 27 that is glued taut over a face of the head portion 2. The top and bottom jaws 24, 26 are connected through a hinge 28.

In use, the wires 6, 7 are connected to monitoring equipment and the instrument is passed along the mother's vagina until the electrodes 9, 10 and head member 2 rest against the scalp of the foetus. The handle 15 is slid down over the trailing end 5 of the main shaft 1, hence pulling the cord 19 which results in the top jaw 24 pivoting relative to the bottom jaw 25 against the resistance of the rubber membrane 27. The membrane 27 is then in a stretched condition. With the head portion 2 pressed against the foetal scalp the plunger 4 is released and the elasticity of the rubber membrane 27 pulls the jaws 24, 25 together, causing the electrodes 9, 10 to penetrate the scalp and closely pass one another, forming a closed loop through the skin. The electrode sheath 11 is constantly in contact with the mother; hence, as soon as the electrodes 9, 10 are fully inserted, monitoring can be performed.

To remove the instrument, the handle 15 is once more moved along the main shaft 1, such that the cord 19 pulls open the jaws 24, 25, uncrosses the electrodes 9, 10 and releases the skin of the baby's scalp. The head portion 2 is gently lifted from the baby's scalp before the handle 15 is once more released, causing the jaws 24, 25 to close. The instrument is then removed from the vagina. Referring to Figs 8 to 21 of the drawings, there is shown a second embodiment of an instrument of the present invention. This embodiment has one electrode 30 in the form of an generally semi-circular needle. The electrode 30 is attached to the top jaw 24 and emerges from the membrane 27 at only one point, this being perforation 26. In the closed jaw position, the tip 31 of the electrode 30 lies adjacent the membrane 27 and, in this way, the tip 31 is shielded during insertion of the instrument into a vagina.

The top jaw 24 is almost entirely encapsulated in an arch-shaped moulded extension 32 of the lower jaw 25, such that the hinge 28 of the jaws 24, 25 is hidden and the leading edge of the instrument is not hingedly movable, thus protecting the patient from further traumas. Backward facing extensions 33 of the lower jaw 25 enclose the cylindrical mould 22 holding the main shaft 1 and shield the emergent cord 19 and the back of the top jaw 24. The extensions 33 are serrated to give a good grip for the operator's fingers when the instrument is in use.

The main shaft 1 terminates at its trailing end 5 in a plunger 4 comprising a handle 15 and an internal slider tube 34, the slider tube 34 being fitted as an extension to the main shaft 1 and being slidable within the handle 15. The cord 19 extends from the bore 8 of the main shaft 1, through a slot 35 in the slider tube 34, to a funnel-shape aperture 36 in the side of the handle 15 wherein the knotted end 18 of the cord 19 is retained. In operation, as for the first embodiment, the handle 15 is moved along the main shaft 1 to open or close the hingedly connected jaws 24,25.

Modifications and improvements may be made without departing from the scope of the invention.

## Claims

1. An instrument for monitoring foetal heart rate comprising a body having a head member (2) for application to the foetal scalp, an electrically conductive needle (9) extending from the head member for penetration of the foetal scalp, a first electrical conductor (6) extending from the needle (9) for connection to monitoring equipment, a second electrical conductor (7) for connection to monitoring equipment, said second conductor being insulated from the first conductor (6) and extending from a terminal (11) external of the body for contacting the mother in use, characterised in that the head member is flexible and the needle is movable therewith, and actuating means (4) are provided for flexing the head member (2).

2. An instrument as claimed in Claim 1, wherein biassing means are provided on the head member (2), to bias said head member (2) towards a preselected attitude.

3. An instrument as claimed in Claim 1 or 2, wherein the biassing means comprises a resilient membrane (27) which engages the head member (2).

4. An instrument as claimed in any one of the preceding Claims, wherein the actuating means (4) is in the form of a telescopic arrangement of members (1,15), the members (1,15) being connected with different regions of the head member (2) for flexing said head member (2). 30

5. An instrument as claimed any one of the preceding Claims, wherein the flexible head member (2) comprises first and second portions (24,25) which are hingedly connected to one another.

6. An instrument as claimed in Claim 5, wherein the needle (9) is arcuate and centred on the axis of the hinge connection (28) between the first and second portions (24,25) of the head member(2).

FIG.3

FIG.1

FIG.2

**FIG.4**

**FIG.5**

**FIG.6**

**FIG.7**

FIG.8

FIG.9

FIG.10

FIG.11

FIG.12

FIG.13

FIG.14

FIG.15

FIG 16

FIG 18

FIG 17

FIG.19

FIG.20

FIG.21